# EUROPEAN PATENT SPECIFICATION

(11) **EP 2 155 000 B1**
(45) Date of publication and mention of the grant of the patent: **18.11.2026**
(21) Application number: 08754267.6
(22) Date of filing: 08.05.2008
(51) Int. Cl.: A23L 29/269, A23L 33/22, A61P 31/00, A61P 35/00, A61P 37/00, A61P 43/00, A23L 2/52, A61K 31/716, A23L 2/39, A23L 5/00, A23P 10/22, A23P 10/40

(54) **PARTICULATE-SOLUBLE GLUCAN PREPARATION**
TEILCHENFÖRMIG LÖSLICHES GLUCANPRÄPARAT
PRÉPARATION DE GLUCANE SOLUBLE DANS DES PARTICULES

(30) Priority: 08.05.2007 US 916690 P
(43) Date of publication of application: 24.02.2010
(73) Proprietor: Biothera, Inc., Eagan, MN 55121 (US)
(72) Inventor: COX, Donald, J., Maple Grove, Minnesota 55408 (US)
(74) Representative: Jacob, Reuben Ellis
(86) International application number: PCT/US2008/005914
(87) International publication number: WO 2008/140744

(56) References cited:
- WO-A1-2005/048735
- WO-A1-2005/120251
- WO-A1-95/30022
- WO-A2-2007/146417
- US-A1- 2003 165 604
- US-A1- 2004 082 539
- US-A1- 2006 172 392
- US-B1- 6 485 945
- JOHANSSON L ET AL: "Hydrolysis of beta-glucan", FOOD CHEMISTRY, ELSEVIER LTD, NL, vol. 97, no. 1, 1 July 2006 (2006-07-01), pages 71 - 79, XP027989343, ISSN: 0308-8146, [retrieved on 20060701]

## Description

### BACKGROUND OF THE INVENTION

The present invention relates to a particulate-soluble β-glucan. More particularly, the present invention relates to an improved method and composition of particulate-soluble β-glucan.

Glucans are generally described as polymers of glucose and are derived from yeast, bacteria, fungi and plants such as oats and barley. Glucans containing a β(1-3)-linked glucopyranose backbone are known to have biological activity, specifically, they have been shown to modulate the immune system and more recently to induce hematopoietic stem and progenitor cell (HSPC) mobilization.

In the past, a high quality soluble β-glucan was expensive to produce. The expense made it cost prohibitive as a nutritional supplement or food ingredient, for example. Therefore, there is a need for an improved soluble β-glucan.

Some previous publications that relate to beta glucan are: WO 2007/146417 A2 which relates to glucan compositions and methods of enhancing CR3 dependent neutrophil-mediated cytotoxicity; US 2006/172392 A1 which relates to water soluble beta-glucan, glucosamine, and N-acetylglucosamine compositions and methods for making the same; WO 95/30022 A1 which relates to the enzyme treatment of glucans; WO 2005/120251 which relates to a method for extracting a cereal constituent; JOHANSSON L ET AL: FOOD CHEMISTRY, ELSEVIER LTD, NL, vol. 97, no. 1, 1 July 2006 (2006-07-01), pages 71-79, XP027989343 which relates to the hydrolysis of beta-glucan; US 2004/082539 A1 which relates to therapeutic uses of beta glucan; US 2003/165604 A1 which relates to products containing beta glucan; US 6 485 945 B1 which relates to polysaccharide compositions and uses thereof; and WO 2005/048735 relates to soluble dietary fibre from oat and barley grains, a method for producing a fraction rich in beta glucan and use of the fraction in foods, pharmaceuticals and cosmetics.

### SUMMARY OF THE INVENTION

According to a first aspect of the invention, there is provided a composition comprising a particulate-soluble β-glucan derived from yeast, the composition being in a dry form, the dry form being capable of re-solubilisation in a liquid that has a pH of 5 or higher, wherein the particulate-soluble β-glucan has a weight average molecular weight of over 1,000,000 Da, wherein the particulate β-glucan is solubilized in an acidic solution at pH 3.6, an elevated temperature of 135°C and pressure of 2.41 bar (35 PSI) for up to 5.5 hours.

### BRIEF DESCRIPTION OF THE DRAWINGS

FIGS. 1-4 are RI traces and data transformations graphs of particulate-soluble β-glucan samples.
FIG. 5 is a graphic representation of TNFa production induced by various β- glucans.

### DETAILED DESCRIPTION OF THE INVENTION

In one example, the starting material is particulate, or insoluble, β-glucan. The particulate β-glucan starting material may range in size from whole glucan particles down to submicron-sized particles. The level of impurities within the starting material may also vary. However, as the level of impurities within the starting material increases, the amount of soluble glucan recovered decreases. One example of suitable starting material is WGP 3-6^{®} from Biothera.

The β-glucan used for the present invention is derived from yeast. The particulate β-glucan is treated with acid under pressure and elevated temperature to produce soluble β-glucan. To begin, pelleted, particulate β-glucan is resuspended and mixed in a sealable reaction vessel in a buffer solution and brought to pH 3.6. Buffer reagents are added such that every liter, total volume, of the final suspension mixture contains about 0.61 g sodium acetate, 5.24 ml glacial acetic acid and 430 g pelleted, particulate β-glucan. The vessel is purged with nitrogen to remove oxygen and increase the pressure within the reaction vessel.

Typically, the pressure inside the vessel is brought to 2.41 bar (35 PSI), and the suspension is heated to about 135°C for up to 5.5 hours. It was found that under these conditions the β-glucan will solubilize. As the temperature decreases from 135°C, the amount of solubilization also decreases.

It should be noted that this temperature and pressure are optimized for the embodiment just described. Optimization and adjustment of temperatures and pressures may be required if any of the reaction conditions and/or reagents are altered.

The increased pressure and temperature imparts advantages over prior art processes for solubilizing β-glucan by virtually eliminating the use of hazardous chemicals from the process. Hazardous chemicals that have previously been used include, for example, flammable VOCs such as ether and ethanol, very strong acids such as formic acid and sulphuric acid and caustic solutions of very high pH. The present process is not only safer, but, by reducing the number of different chemicals used and the number of steps involved, is more economical.

An additional advantage of the present process is that the length of the above treatment controls the molecular weight of the resulting particulate-soluble β-glucan. As the term is used throughout, "particulate-soluble β-glucan" may be in any of the soluble, particulate or re-solubilized forms. The length of treatment time is inversely related to the resulting molecular weight size. In other words, as the treatment time increases, the resulting average molecular weight of the particulate-soluble β-glucan decreases. This principal is illustrated in the following.

Four samples were collected from a batch of particulate-soluble β-glucan suspension at various time-points (60, 90, 120 and 240 min.) during the above-described process. The molecular weights of the samples were determined by size exclusion chromatography against dextran molecular weight markers. The results are summarized in Table 1.

**TABLE 1**

| **Sample Time** | **Mn*** | **Mw**** | **Mz***** | **Hexose Conc.** |
|---|---|---|---|---|
| 60 min. | 5,500 | 352,000 | 4,250,000 | 5.16 mg/ml |
| 90 min. | 8,500 | 1,180,000 | 5,430,000 | 12.33 mg/ml |
| 120 min. | 6,600 | 354,000 | 2,730,000 | 11.26 mg/ml |
| 240 min. | 5,100 | 56,500 | 362,000 | ≈25 mg/ml |

| | | | | |
|---|---|---|---|---|
| *number average molecular weight calculated by: $\overline{M} n = \frac{{\sum }_{i} {N}_{i} {M}_{i}}{{\sum }_{i} {N}_{i}}$ **weight average molecular weight calculated by: $\overline{M} w = \frac{{\sum }_{i} {N}_{i} {M}_{i}^{2}}{{\sum }_{i} {N}_{i} {M}_{i}}$ where *Nᵢ* is the number of molecules of molecular weight *Mᵢ.* ***z-averaged molecular weight, which is based on the volume of the polymer chains. | | | | |

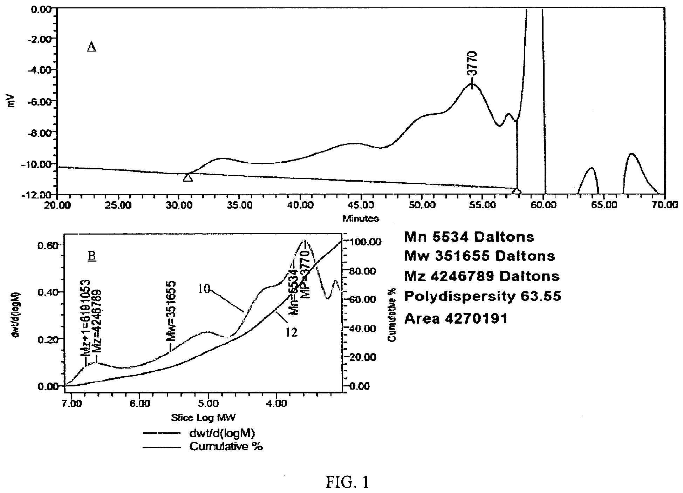

As evidenced by the above data, the particulate β-glucan slowly begins to breakdown with the largest average size occurring at about 90 min. After the 90 min. time-point, the average size decreases. Time-point specific data is shown in FIGS. 1-4.

FIG. 1 includes panels A and B. Panel A is a refractive index (RI) trace of the 60 min. sample. In panel B, a section of the data generated in panel A was transformed and plotted on the graph shown. Curve 10 represents the transformed raw data. Curve 12 represents the cumulative percentage of specific molecular weights. Molecular weight was determined by a standard curve generated with dextran of known size (data not shown).

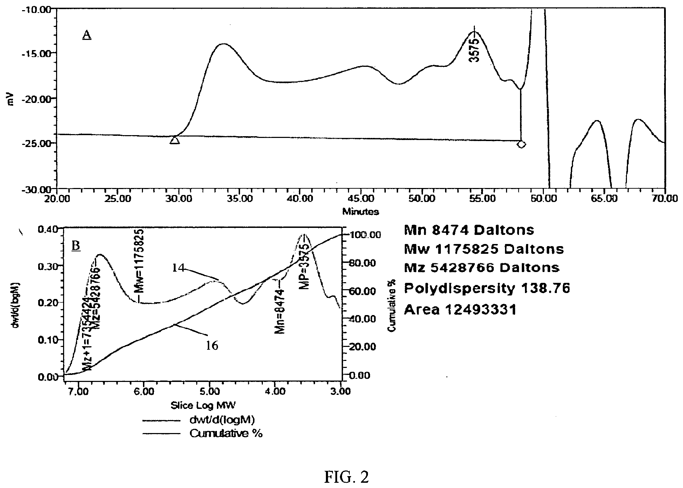

Data generated from the 90 min. sample is shown in FIG. 2. Panel A and panel B, with curves 14 and 16, correspond to the panels A and B of FIG. 1.

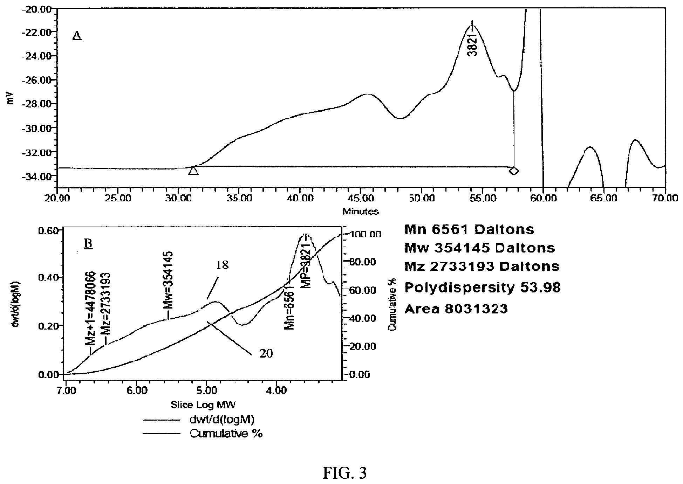

Data generated from the 120 min. sample is shown in FIG. 3. Panel A and panel B, with curves 18 and 20, correspond to the panels A and B of FIG. 1.

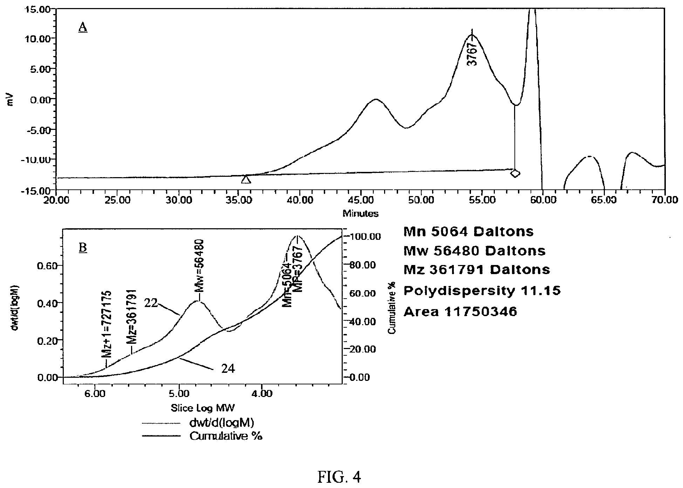

Data generated from the 240 min. sample is shown in FIG. 4. Panel A and panel B, with curves 22 and 24, correspond to the panels A and B of FIG. 1.

Specific sizes of particulate-soluble glucan may be more useful for specific applications. For example, when applied topically, molecules of smaller molecular weight may better penetrate skin. Larger molecular weight molecules, on the other hand, may be better suited for oral administration. Therefore, the ability to easily control the average size of the glucan molecules offers a significant competitive advantage.

The exact duration of heat treatment is typically determined experimentally by sampling reactor contents. Various tests may be performed such as gel permeation chromatography (GPC) analyses. The process maximizes the yield of particulate-soluble material that meets specific specification profiles and impurity levels. Once the β-glucan is solubilized, the mixture is cooled to stop the reaction.

The particulate-soluble β-glucan may be washed and utilized at this point, however, for pharmaceutical applications further purification is typically performed. Any combination of one or more of the following steps may be used to further purify and/or clarify the particulate-soluble β-glucan. Other means known in the art may also be used if desired. Suitable means include, for example, centrifugation or continuous-flow centrifugation, which eliminates a significant amount of lipid impurities.

In some instances, the inclusion of lipids within the particulate-soluble β-glucan may increase its effectiveness against certain infectious diseases or enhancing the immune system. The process presented here provides a means to effectively control and vary the amount of lipid in the particulate-soluble β-glucan composition.

Alternatively or in addition to, the particulate-soluble β-glucan may be filtered. In one embodiment, the material is filtered through a 0.2 µm filter.

Chromatography may also be used for purification. The particulate-soluble β-glucan may be conditioned at some point in preparation for chromatography. For example, if a chromatographic step includes hydrophobic interaction chromatography (HIC), the particulate-soluble β-glucan can be conditioned to the appropriate conductivity and pH with a solution of ammonium sulphate and sodium acetate. A suitable solution is 3.0 M ammonium sulfate, 0.1 M sodium acetate, which is used to adjust the pH to 5.5.

Another chromatographic step that may be utilized is gel permeation chromatography (GPC). Multiple chromatography cycles may be needed to ensure that the load does not exceed the capacity of the column.

At this point, the particulate-soluble β-glucan is purified and ready for use. The particulate-soluble β-glucan that is generally collected has an average molecular weight >1,000,000 Da, with a high polydispersity. An advantage of this particulate-soluble β-glucan product, especially in the high molecular weight ranges such as over a million Daltons (Da), is that the product can be spray dried to a powder form and then subsequently re-solubilized in solutions of pH about 5 and higher-essentially from weakly acidic to strongly basic. In addition, re-solubilization occurs at temperatures as low as about room temperature (~25°C). Depending on the final concentration after re-solubilization and the solvent used, the time and temperature requirements for re-solubilization will vary.

The product's ability to re-solubilize makes it ideal for creating an instantized form of β-glucan suitable for, for example, instant drink powders. Briefly, particulate-soluble β-glucan in dry form is re-wet to achieve a moisture content of about 9%-12%, which forms an agglomerate. A surfactant, such as lecithin, is added to the agglomerate via a two-fluid nozzle. The concentration of surfactant will vary depending on the surfactant used and the conditions and solvent into which the product will be finally dissolved. The agglomerate is then run through three stages of a fluid bed drier. The first stage heats the product, the second stage conditions the product and the third stage cools the product. The resulting product is sifted to form the instantized product.

Another way to form an instantized product is through the addition of a second dry-form soluble ingredient such as table sugar or salt (NaCl). In one example, 200 mg of powdered, particulate-soluble β-glucan and about 0.5 to about 1.0 g of sucrose crystals were mixed and ground in a mortor bowl for about 2-3 minutes. The mixture was added to 500 mis of room temperature water and shook vigorously. The particulate-soluble β-glucan re-solubilized. An identical experiment was carried out with NaCl with the same result.

The particulate-soluble β-glucan product, whether in its solubilized, powdered or re-solubilized form, may be used for any of a number of applications. Particulate-soluble β-glucan is ideal for use as a nutritional supplement, a food ingredient, a cosmetic ingredient and a pharmaceutical product for use in humans and agricultural/companion/exotic animals.

Typical foods in which the dry or wet forms of particulate-soluble β-glucan may be added include, for example, cereals and cereal products, baked goods and baking mixes, beverages and beverage bases, dairy product analogs such as soy milk, milk and milk products, plant protein products, processed fruits and fruit juices, soft candy, soup and soup mixes, yogurt, bottled water and drinks, nutrition bars, etc. Various masking agents and other additives may be added to improve the flavor and texture if necessary.

In its liquid form, the particulate-soluble β-glucan can be used to make edible films. A thin layer of liquid product is dried by any of a number of means known in the art. Additives may be added to the liquid product to create films of various textures or to make them more or less pliable. The films are useful as carriers for pharmaceutical or supplement products. Here again, various masking agents can be added to improve the flavor of the film.

The particulate-soluble β-glucan product may also be more efficacious than prior art particulate β-glucan. For example, the product may be more effective against infectious diseases or cancer. Increased efficacy may be in terms of better uptake, better immune system enhancement, decreased dosing regiments, etc.

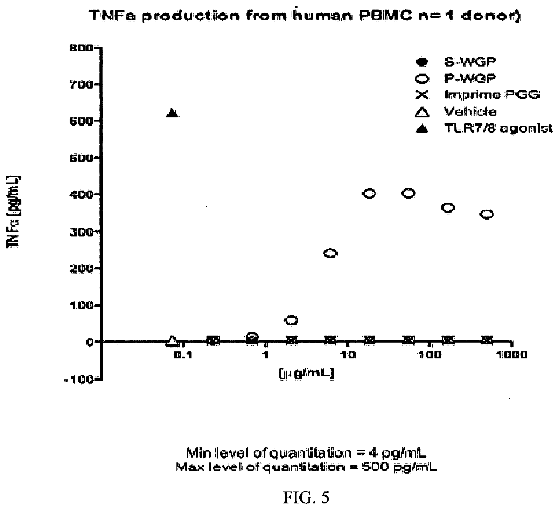

To this end, in vitro studies showed that the particulate-soluble β-glucan does not induce TNFα from human peripheral blood mononuclear cells (PBMC) at concentrations up to 300 µg/mL. Specifically, human PBMC were cultured in cRPMI medium containing 5% heat-inactivated FCS. Cells were stimulated with various beta glucans (all endotoxin free), TLR7/8 agonist, or vehicle. The beta glucans were used at various concentrations. After overnight incubation at 37°C, 5% CO₂, cell-free supernatants were collected, stored at -20°C until cytokine analysis by ELISA. The results from three replicates are shown in FIG. 5. As evidenced by the graph, whole glucan particles stimulate TNFα production, while the particulate-soluble product does not. Because TNFα is a major cytokine involved in an inflammatory response, the particulate-soluble product is not expected to induce an inflammatory response.

## Claims

1. A composition comprising a particulate-soluble β-glucan derived from yeast, the composition being in a dry form, the dry form being capable of re-solubilisation in a liquid that has a pH of 5 or higher, wherein the particulate-soluble β-glucan has a weight average molecular weight of over 1,000,000 Da, wherein the particulate β-glucan is solubilized in an acidic solution at pH 3.6, an elevated temperature of 135°C and pressure of 2.41 bar (35 PSI) for up to 5.5 hours.

## Patentansprüche

1. Zusammensetzung, die ein aus Hefe gewonnenes, teilchenlösliches β-Glucan umfasst, wobei die Zusammensetzung in trockener Form vorliegt, wobei die trockene Form in der Lage ist, in einer Flüssigkeit mit einem pH-Wert von 5 oder höher resolubilisiert zu werden, wobei das teilchenlösliche β-Glucan ein gewichtsmittleres Molekulargewicht von über 1.000.000 Da aufweist, wobei das teilchenlösliche β-Glucan in einer sauren Lösung bei einem pH-Wert von 3,6, einer erhöhten Temperatur von 135 °C und einem Druck von 2,41 bar (35 PSI) bis zu 5,5 Stunden lang solubilisiert wird.

## Revendications

1. Composition comprenant un β-glucane particulaire soluble dérivé de levure, la composition se présentant sous forme sèche, la forme sèche pouvant être resolubilisée dans un liquide ayant un pH égal ou supérieur à 5, où le β-glucane particulaire soluble présente un poids moléculaire moyen en poids supérieur à 1 000 000 Da, où le β-glucane particulaire est solubilisé dans une solution acide à un pH de 3,6, à une température élevée de 135°C et sous une pression de 2,41 bar (35 PSI) pendant une durée pouvant aller jusqu'à 5,5 heures.
